# EUROPEAN PATENT APPLICATION

(11) **EP 3 670 516 A1**
(43) Date of publication of application: **24.06.2020**
(21) Application number: 19209377.1
(22) Date of filing: 15.11.2019
(51) Int. Cl.: C07D 493/04, C08F 136/20

(54) **ISOSORBIDE BASED PHOTOCURABLE COMPOSITION AND METHOD FOR PREPARING THE SAME**

(30) Priority: 21.12.2018 KR 20180166945
(71) Applicant: Jeil Chemical Corporation Limited, Ulsan 44992 (KR)
(72) Inventor: KWON, Sung-Hun, 46232 Busan (KR); JUNG, Sin-Hye, 46232 Busan (KR); JUNG, Woo-Sun, 46232 Busan (KR)
(74) Representative: AWA Sweden AB

(57) **Abstract**

An isosorbide-based photocurable composition contains a compound. The compound contains isosorbide in a main chain thereof, and contains an acrylate group at an terminal thereof. The compound is represented by a following Chemical Formula 1 or Chemical Formula 2: 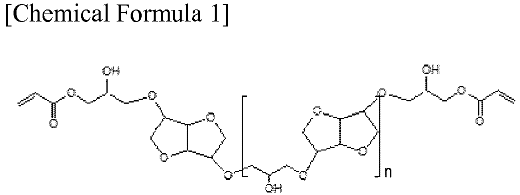 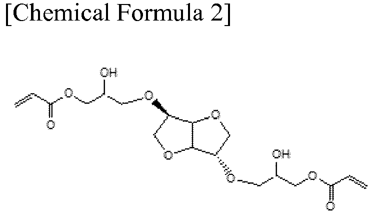 wherein in the Chemical Formula 1, n denotes an integer of 1 to 1000.

## Description

### CROSS-REFERENCE TO RELATED APPLICATIONS

This application claims the priority of Korean Patent Application No. 10-2018-0166945 filed on December 21, 2018 in the Korean Intellectual Property Office, the disclosure of which is hereby incorporated by reference in its entirety.

### BACKGROUND

### 1. Technical Field

The present disclosure relates to an isosorbide-based photocurable composition obtained by synthesizing to an isosorbide derivative with acrylic acid and having various applications as photocurable materials for 3D printing, building paints, and coatings, and to a preparation method thereof.

### 2. Description of the Related Art

Among an epoxy, bisphenol A type epoxy being economic and having excellent properties occupies more than 80% of all of epoxies. The bisphenol A type epoxy has wide applications including an inner coating of a food and beverage container, an inner coating of a water purification plant, and a construction coating. Therefore, the human may contact the bisphenol A type epoxy easily in everyday life.

However, as is well known, the bisphenol A (BPA) is an environmental hormone with a structure similar to a female hormone estrogen. Thus, the bisphenol A (BPA) is considered as a very strong physiologically toxic substance. Thus, our society prohibits its use in contact with humans in countries around the world.

In particular, developed countries including EU enforce Directives and Regulations on the Use of Harmful Substances to prohibit the use of the compounds harmful to the environment directly or indirectly regulate the trade thereof. This trend is expected to bn intensified. Korea designated the bisphenol A (BPA) as a restricted substance for household goods. Developed countries are investing heavily in the research of BPA-free bio epoxy resin, which will be a core product in a future epoxy market. However, domestic research is insufficient due to lack of demand and poor technology.

In one example, an isosorbide based bio-epoxy resin is free of BPA and thus is bio-friendly compared to the existing BPA epoxy. Further, the isosorbide-based bio-epoxy has not only non-yellowing properties but also excellent physical properties such as mechanical strength and thermal properties.

However, during the synthesis process for isosorbide-based bio-epoxy, it may be difficult to control equivalent due to a large amount of produced oligomer. Further, it may be difficult to mass-produce the isosorbide-based bio-epoxy having a constant physical properties. Further, the physical properties of the isosorbide-based bio-epoxy product has EEW (epoxy equivalent weight) between 220 and 176 which is higher compared to the isosorbide molecular weight, such that the oligomer content is high. This causes deterioration in physical properties.

Therefore, intensive research on the BPA-free bio-epoxy resin is needed to develop a future epoxy industry and secure a core material.

### SUMMARY

A purpose of the present disclosure is to provide an isosorbide-based photocurable composition which may replace the BPA based epoxy and may be applied to an eco-friendly biomaterial required by a market.

Further, another purpose of the present disclosure is to provide an isosorbide-based photocurable composition that may be applied to a non-toxic building floor paint, an environmentally-friendly photocurable coating, and a 3D printing photocurable material.

Purposes of the present disclosure are not limited to the above-mentioned purpose. Other purposes and advantages of the present disclosure as not mentioned above may be understood from following descriptions and more clearly understood from embodiments of the present disclosure. Further, it will be readily appreciated that the purposes and advantages of the present disclosure may be realized by features and combinations thereof as disclosed in the claims.

In a first aspect, there is provided an isosorbide-based photocurable composition containing a compound, wherein the compound contains isosorbide in a main chain thereof, and contains an acrylate group at an terminal thereof, wherein the compound is represented by a following Chemical Formula 1 or Chemical Formula 2: 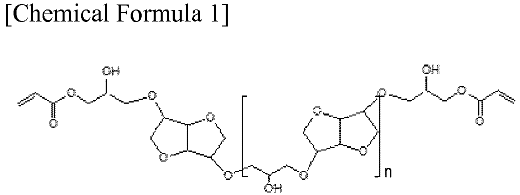 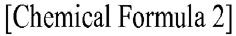 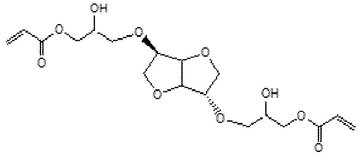 wherein in the Chemical Formula 1, n denotes an integer of 1 to 1000.

In one embodiment, the compound represented by the Chemical Formula 1 or Chemical Formula 2 has a viscosity of 80,000 to 100,000 cps at a temperature 25 ± 5 °C.

In another aspect, there is provided a method for preparation of an isosorbide-based photocurable composition, the method comprising: (a) mixing a first compound and an acrylic compound with each other under presence of catalyst to form a mixture and stirring the mixture at 80 to 100 °C for a synthesis reaction; (b) adding a polymerization inhibitor to a product from the synthesis reaction; (c) terminating the synthesis reaction when an acid value of a product resulting from the step (b) is 2 mgKOH/g or smaller, wherein the first compound includes diglycidyl ether of isosorbide (DGEI) containing isosorbide in a main chain thereof, and containing a glycidyl group at a terminal thereof, and being represented by a following Chemical Formula 3 or Chemical Formula 4: 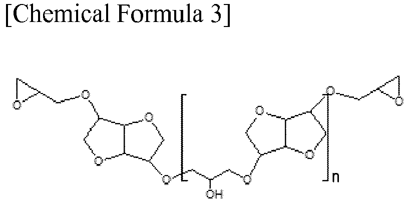 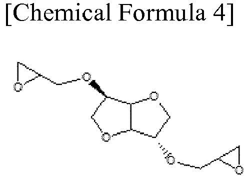 wherein n in the Chemical Formula 3 denotes an integer of 1 to 1000.

In one embodiment, in the (a) step, the catalyst includes at least one selected from a group consisting of an amine based catalyst, a tin based catalyst and a bismuth based catalyst.

In one embodiment, in the (a) step, 100 parts by weight of the first compound is mixed with 0.1 to 10 parts by weight of the acrylic compound, wherein the acrylic compound contains at least one of acrylic acid or methacrylic acid.

In one embodiment, in the step (b), 500 to 1000 ppm of the polymerization inhibitor is added to the product, wherein the polymerization inhibitor includes hydroquinone monomethyl ether (HQMME).

Effects of the present disclosure are as follows but are not limited thereto.

The isosorbide-based photocurable composition according to the present disclosure is prepared by reacting diglycidyl ether of isosorbide (DGEI) synthesized from isosorbide as corn-based 100% natural biomaterial with an acrylic compound. Accordingly, the prepared photocurable composition is more environmentally friendly than the BPA epoxy and has non-yellowing properties. In addition, mechanical strength and thermal properties of the isosorbide-based photocurable composition are excellent.

In addition, the isosorbide-based photocurable composition according to the present disclosure may be used for eco-friendly non-toxic building floor coatings, eco-friendly solvent-free photocurable coatings, and 3D printing photocurable materials.

In addition to the effects described as above, specific effects of the present disclosure will be described together with the detailed description for carrying out the present disclosure.

### BRIEF DESCRIPTION OF DRAWINGS

FIG. 1 shows GPC results of diglycidyl ether of isosorbide (DGEI) (left) and DGEI (right) with an acrylate group.
FIG. 2 shows a IR analysis of DGEI with an acrylate group in accordance with the present disclosure.

### DETAILED DESCRIPTIONS

For simplicity and clarity of illustration, elements in the figures are not necessarily drawn to scale. The same reference numbers in different figures denote the same or similar elements, and as such perform similar functionality. Furthermore, in the following detailed description of the present disclosure, numerous specific details are set forth in order to provide a thorough understanding of the present disclosure. However, it will be understood that the present disclosure may be practiced without these specific details. In other instances, well-known methods, procedures, components, and circuits have not been described in detail so as not to unnecessarily obscure aspects of the present disclosure.

Examples of various embodiments are illustrated and described further below. It will be understood that the description herein is not intended to limit the claims to the specific embodiments described. On the contrary, it is intended to cover alternatives, modifications, and equivalents as may be included within the spirit and scope of the present disclosure as defined by the appended claims.

The terminology used herein is for the purpose of describing particular embodiments only and is not intended to be limiting of the present disclosure. As used herein, the singular forms "a" and "an" are intended to include the plural forms as well, unless the context clearly indicates otherwise. It will be further understood that the terms "comprises", "comprising", "includes", and "including" when used in this specification, specify the presence of the stated features, integers, operations, elements, and/or components, but do not preclude the presence or addition of one or more other features, integers, operations, elements, components, and/or portions thereof. As used herein, the term "and/or" includes any and all combinations of one or more of the associated listed items. Expression such as "at least one of' when preceding a list of elements may modify the entire list of elements and may not modify the individual elements of the list.

It will be understood that when an element or layer is referred to as being "connected to", or "coupled to" another element or layer, it can be directly on, connected to, or coupled to the other element or layer, or one or more intervening elements or layers may be present. In addition, it will also be understood that when an element or layer is referred to as being "between" two elements or layers, it can be the only element or layer between the two elements or layers, or one or more intervening elements or layers may also be present.

Unless otherwise defined, all terms including technical and scientific terms used herein have the same meaning as commonly understood by one of ordinary skill in the art to which this inventive concept belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

Hereinafter, an isosorbide-based photocurable composition and a preparation method thereof according to some embodiments of the present disclosure will be described.

The isosorbide is a 100% biomass material based on corn, and is produced by extracting starch from corn and then treating the starch with glucose and sorbitol. Plastics made of isosorbide are unlikely to be toxic and decompose, unlike plastics based on conventional petrochemicals. The plastic made of isosorbide has excellent properties such as transparency and surface hardness.

The present disclosure provides a biomass-based photocurable composition produced using diglycidyl ether of isosorbide (DGEI) having isosorbide as biomass material and more environmentally friendly than the conventional BPA epoxy and having non-yellowing properties and excellent mechanical strength and provides a preparation method thereof.

### Isosorbide-based photocurable composition

The isosorbide is a bicyclic compound of a diol containing two fused furan rings and a heterocyclic compound containing oxygen. The isosorbide is a 100% natural biomaterial made from corn. The isosorbide is D-sorbitol obtained by extracting starch from corn and by contacting hydrogenation of D-glucose as produced by hydrolysis of the starch. This isosorbide is a material from which biodegradable derivatives of various functions may be obtained.

In accordance with the present disclosure, an isosorbide-based photocurable composition contains a compound represented by a following Chemical Formula 1 or Chemical Formula 2: 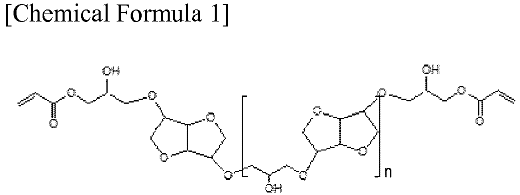 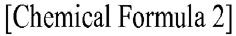 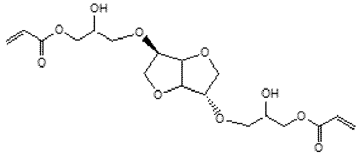 where n in the Chemical Formula 1 is an integer from 1 to 1000.

Referring to the Chemical Formula 1 and Chemical Formula 2, the compound contains an isosorbide in the main chain thereof and an acrylate group at each of both terminals thereof.

The isosorbide-based compound represented by the Chemical Formula 1 or Chemical Formula 2 has a viscosity of 80,000 to 100,000 cps at 25 ± 5 °C. The isosorbide-based compounds represented by the Chemical Formula 1 or Chemical Formula 2 have the low viscosity and heat resistance. Further, the compound represented by the Chemical Formula 1 or Chemical Formula 2 has an acid value of 1 to 2 mgKOH/g and a color value of 0.4 to 0.7 G and a specific gravity of 1.0 to 1.3.

### Preparation method of isosorbide-based photocurable composition

A preparation method of the isosorbide-based photocurable composition in accordance with the present disclosure may include (a) mixing a first compound and an acrylic compound with each other under presence of catalyst to form a mixture and stirring the mixture at 80 to 100 °C for a synthesis reaction; (b) adding a polymerization inhibitor to a product from the synthesis reaction; and (c) terminating the synthesis reaction when an acid value of a product resulting from the step (b) is 2 mgKOH/g or smaller,

First, the first compound and the acrylic compound are introduced into a reactor in which the catalyst is present, thereby to form a mixture. Then, the mixture is stirred at 80 to 100 °C for a synthesis reaction.

The catalyst is added to accelerate the reaction. In the (a) step, the catalyst includes at least one selected from a group consisting of an amine based catalyst, a tin based catalyst and a bismuth based catalyst. For example, the catalyst may include at least one selected from a group consisting of DABCO(1,4-diazabicyclo[2.2.2]octane), DBTDL(dibutyltin dilaurate), stannous octaoate(tin 2-ethylhexanoate), and bismuth octoate. In one example, the DBTDL is a lemon yellow liquid with high catalytic activity, which makes it easy to control a reaction rate.

A content of the catalyst may be contained in an amount of 0.1 to 5 parts by weight based on 100 parts by weight of the first compound. When the content of the catalyst is smaller than 0.1 parts by weight, the reaction rate is low. On the contrary, when the content thereof exceeds 5 parts by weight, a by-product may be generated due to rapid heat generation.

The first compound includes liquid-phase diglycidyl ether of isosorbide (DGEI) containing isosorbide in a main chain thereof, and containing a glycidyl group at a terminal thereof, and being represented by a following Chemical Formula 3 or Chemical Formula 4: 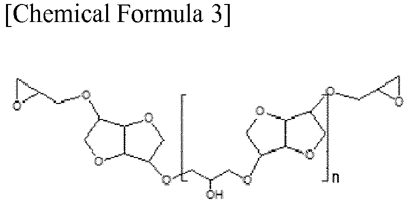 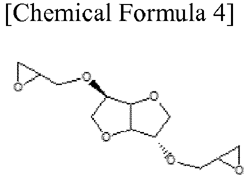 wherein n in the Chemical Formula 3 denotes an integer of 1 to 1000.

To couple the acrylate group to each of both terminals of the first compound, an acrylic compound is added thereto. The acrylate group has a structure which may be hardened when being subjected to light irradiation. The acrylate group is highly reactive because it is activated by light irradiation.

The acrylic compound may include a compound having one or more unsaturated carboxyl groups. For example, the acrylic compound may include acrylic acid and/or methacrylic acid.

It is preferable to add 0.1 to 10 parts by weight of the acrylic compound to 100 parts by weight of the first compound. When a content of the acrylic compound is smaller than 0.1 part by weight, a synthesis of the acrylate group is not performed properly. On the contrary, when the content thereof exceeds 10 parts by weight, it is difficult to synthesize a compound having a desired structure.

The stirring step may be preferably carried out at 80 to 100 °C. When the stirring temperature is outside this temperature range, the synthesis may not be sufficient.

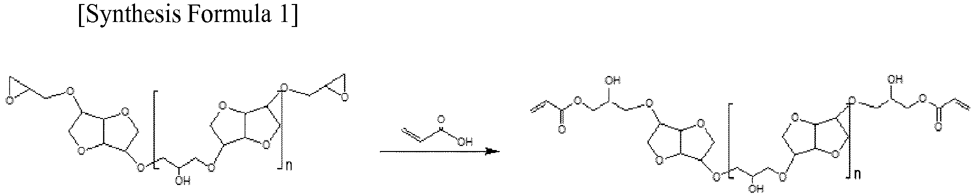

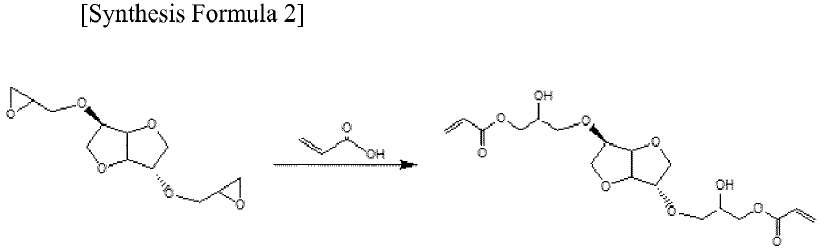

During the stirring step, the acrylate group is synthesized to the terminal of DGEI represented by the Chemical Formula 3 or Chemical Formula 4. In this connection, while the remaining DGEI and acrylate are being synthesized with each other, the polymerization inhibitor may be added to prevent polymerization of the already synthesized DGEI.

The polymerization inhibitor may include hydroquinone monomethyl ether (HQMME), and may inhibits polymerization. The polymerization of the produced DEGI acrylate does not occur until the polymerization inhibitor has been exhausted out. The polymerization inhibitor may contain sulfur and benzoquinone in addition to HQMME.

A content of the polymerization inhibitor may be adjusted based on the input content of DGEI. The polymerization inhibitor may preferably be added at a content of 500 to 1000 ppm.

In addition, 0.01 to 0.1 parts by weight of tetrabutylphosphonium malonate may further be added while the polymerization inhibitor is added. When the tetrabutylphosphonium malonate is further added, excellent mechanical properties of the composition may be exhibited.

Subsequently, the reaction may be terminated when an acid value of a product resulting from the step (b) is 2 mgKOH/g or smaller.

The synthesis reaction may be terminated when an acid value of a product resulting from the step (b) is 2 mgKOH/g or smaller. Then, it may be confirmed using Fourier transform Infrared spectroscopy (FT-IR) that the epoxy peak disappears and an acrylate double bond peak appears.

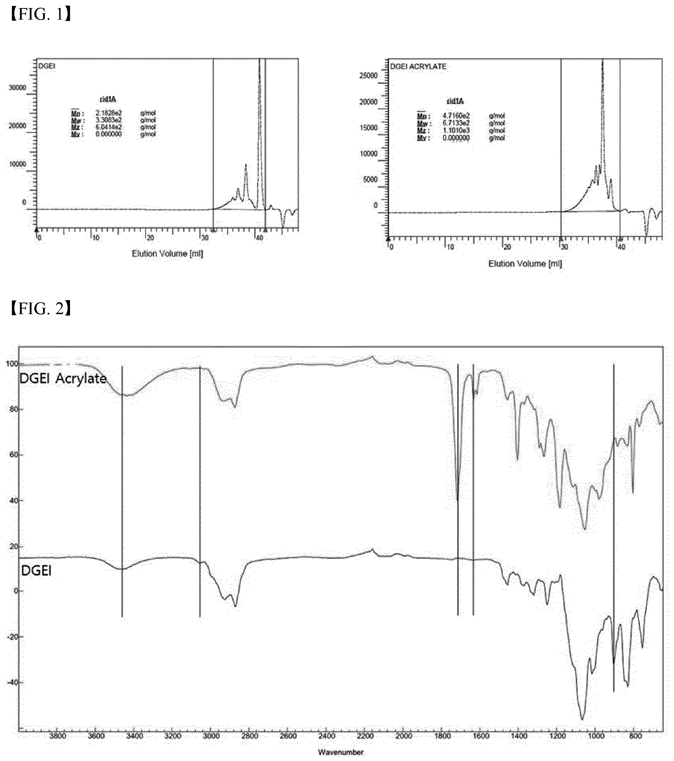

FIG. 1 shows GPC results of diglycidyl ether of isosorbide (DGEI) (left) and DGEI (right) with the acrylate group.

Referring to FIG. 1, before synthesis of the acrylic acid with DGEI, a molecular weight of DGEI was 330g/mol. After synthesizing the acrylic acid with DGEI, the molecular weight thereof was 670 g/mol which was about 2 times higher than 330 g/mol. This may be due to partial epoxy self-condensation in the synthesis process which may occur due to properties of the epoxy acrylate.

FIG. 2 shows the IR analysis of DGEI with an acrylate group in accordance with the present disclosure. The FT-IR was analyzed before and after synthesizing the acrylic acid with the DGEI.

Referring to FIG. 2, a -OH peak at 3438 cm⁻¹ was increased due to the effect of -OH generated when the epoxy group of the DGEI was synthesized with the acrylic acid. Further, a oxirane C-H peak of DGEI at 3057cm⁻¹ and a C-O oxirane peak thereof at 907cm⁻¹ disappeared as they were acrylated in the DGEI.

Further, it was confirmed that the C = O peak at 1720cm⁻¹ and a C = C acrylate peak at 1635cm⁻¹ appeared.

As a result, the epoxy in DEGI disappeared and the peak of the acrylate group occurred, thereby confirming that DGEI was efficiently synthesized with acrylate.

As such, according to the present disclosure, synthesizing the acrylic compound with the biomaterial DGEI may allow the isosorbide-based photocurable composition to be synthesized in a relatively simple process. The synthetic isosorbide-based photocurable compositions may be expected to have the same physical properties as the conventional BPA epoxy. Therefore, the synthesized isosorbide-based photocurable composition is an eco-friendly composition that may replace the existing BPA epoxy and may be mass-produced. Further, the photocurable composition in accordance with the present disclosure is highly applicable to environmentally friendly materials such as molding compositions for interior materials, coating compositions, flooring materials, internal coating liquids for water pipes, and 3D photocurable materials.

Further, the photocurable composition in accordance with the present disclosure is applicable to a food packaging, a sealing container, and a heavy-electrical insulator that require water resistance and weather resistance.

Present examples of the isosorbide-based photocurable composition and the preparation method thereof are as follows.

### 1. Preparation of isosorbide-based photocurable composition

### Present Example 1

A DBTDL catalyst (Aldrich) was introduced into a reactor. 0.1 part by weight of acrylic acid (TCI) was added to 100 parts by weight of the first compound represented by the Chemical Formula 3 (n = 2). Then, the mixture was stirred at 95 ° C for 8 hours. Subsequently, 1000 ppm of HQMME (Aldrich) as the polymerization inhibitor was added to the reactor. Samples were taken every 2 hours. Then, the reaction was terminated at an acid value of 2 mgKOH/g or smaller.

### Present Example 2

A composition was prepared under the same conditions as in Present Example 1, except that the first compound represented by the Chemical Formula 4 was used instead of the compound expressed by the Chemical Formula 3.

### Present Example 3

A composition was prepared under the same conditions as in Present Example 1, except that 10 parts by weight of acrylic acid was added.

### Present Example 4

A composition was prepared under the same conditions as in Present Example 2, except that the first compound represented by the Chemical Formula 4 was used instead of the compound expressed by the Chemical Formula 3.

### Present Example 5

A composition was prepared under the same conditions as in Present Example 1, except that when HQMME (Aldrich) was added, 0.01 part by weight of tetrabutylphosphonium malonate (Sigma Aldrich) was added to 100 parts by weight of the first compound.

### Comparative Example 1

A composition containing a compound represented by the Chemical Formula 3 (n = 2) was prepared.

### Comparative Example 2

A composition was prepared under the same conditions as in Present Example 1, except that 0.01 part by weight of acrylic acid (TCI) was added.

### Comparative Example 3

A composition was prepared under the same conditions as in Present Example 1, except that 15 parts by weight of acrylic acid (TCI) was added.

### 2. Method for evaluating properties, and results thereof

### 1) Brine resistance, KS D 9502

- A test method sprayed salt solution to a specimen made of each of the compositions for a certain period of time and measure whether the specimen is free of rust, blistering and cracking.

### 2) Water resistance, KS M ISO 2812-2

- A standard specimen in which each composition coats a test plate was partially immersed in water at room temperature such that 1/4 of the specimen was not immersed. In a predefined duration, the specimen was taken out and dried, and then checked in terms of abnormalities such as blistering, rust, color change and loss of adhesion.

### 3) Hardness, ASTM D 2240

- A thickness of the specimen was at least 6 mm. A hardness thereof was measured three times under a load of about 5 kg (49 N) using a Shore D type hardness tester, and then the measurements were averaged.

### 4) Tensile strength, KSM 3015 standard

- A test method measured a tensile strength of a specimen made by 3D printing each composition based on a KSM 3015 standard.

**Table 1**

| | Brine resistance | Water resistance | Shore hardness | Tensile strength(MPa) | Viscosity(25°C,cps) |
|---|---|---|---|---|---|
| Present Example 1 | No crack | Normal | 85D | 65 | 80,000 |
| Present Example 2 | No crack | Normal | 83D | 68 | 80,000 |
| Present Example 3 | No crack | Normal | 83D | 68 | 81,000 |
| Present Example 4 | No crack | Normal | 85D | 75 | 80,000 |
| Present Example 5 | No crack | Normal | 92D | 90 | 80,000 |
| Comparative Example 1 | Crack | Rust, color change | 60D | 42 | 95,000 |
| Comparative Example 2 | No crack | Rust | 60D | 65 | 100,000 |
| Comparative Example 3 | Crack | Color change | 60D | 40 | 100,000 |

Referring to the results of Table 1, the photocurable composition according to the present disclosure shows excellent results in terms of brine resistance, water resistance, shore hardness (80D to 95D) and tensile strength (65 to 90MPa).

To the contrary, it may be confirmed that when the acrylate is not synthesized with DGEI or when the amount of acrylic acid as added is not in a range of 0.1 to 10 parts by weight, cracks occur in the cured product or mechanical properties thereof are degraded.

Although the present disclosure has been described with reference to the drawings and embodiments as exemplified above, the present disclosure is not limited to the embodiments and the drawings disclosed herein. It is obvious that various modifications may be made thereto by a person skilled in the art within the scope of the present disclosure. In addition, it should be appreciated that effects to be achieved from configurations of the present disclosure as not expressly mentioned may be acknowledged.

## Claims

1. An isosorbide-based photocurable composition containing a compound, wherein the compound contains isosorbide in a main chain thereof, and contains an acrylate group at an terminal thereof, wherein the compound is represented by a following Chemical Formula 1 or Chemical Formula 2: 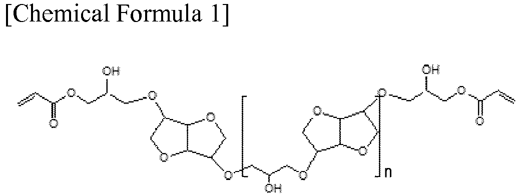 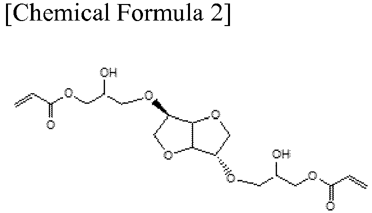 wherein in the Chemical Formula 1, n denotes an integer of 1 to 1000.

2. The isosorbide-based photocurable composition of claim 1, wherein the compound represented by the Chemical Formula 1 or Chemical Formula 2 has a viscosity of 80,000 to 100,000 cps at a temperature 25 ± 5 °C.

3. A method for preparation of an isosorbide-based photocurable composition, the method comprising:
(a) mixing a first compound and an acrylic compound with each other under presence of catalyst to form a mixture and stirring the mixture at 80 to 100 °C for a synthesis reaction;
(b) adding a polymerization inhibitor to a product from the synthesis reaction; and
(c) terminating the synthesis reaction when an acid value of a product resulting from the step (b) is 2 mgKOH/g or smaller,
wherein the first compound includes diglycidyl ether of isosorbide (DGEI) containing isosorbide in a main chain thereof, and containing a glycidyl group at a terminal thereof, and being represented by a following Chemical Formula 3 or Chemical Formula 4: 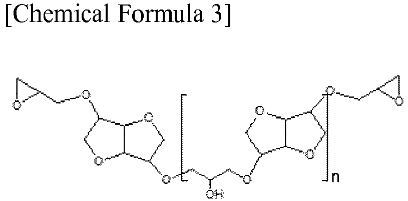 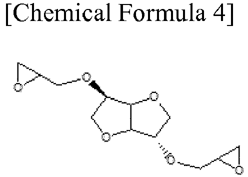 wherein n in the Chemical Formula 3 denotes an integer of 1 to 1000.

4. The method of claim 3, wherein in the (a) step, the catalyst includes at least one selected from a group consisting of an amine based catalyst, a tin based catalyst and a bismuth based catalyst.

5. The method of claim 3, wherein in the (a) step, 100 parts by weight of the first compound is mixed with 0.1 to 10 parts by weight of the acrylic compound, wherein the acrylic compound contains at least one of acrylic acid or methacrylic acid.

6. The method of claim 3, wherein in the step (b), 500 to 1000 ppm of the polymerization inhibitor is added to the product, wherein the polymerization inhibitor includes hydroquinone monomethyl ether (HQMME).
